Europäisches Patentamt

European Patent Office

Office européen des brevets

(11) Publication number: **0 490 305 A2**

## EUROPEAN PATENT APPLICATION

(21) Application number: **91121029.2**

(22) Date of filing: **07.12.91**

(51) Int. Cl.5: **A61K 9/00, A61K 9/52**

(30) Priority: **12.12.90 JP 419242/90**

(43) Date of publication of application:
**17.06.92 Bulletin 92/25**

(84) Designated Contracting States:
**AT BE CH DE DK ES FR GB GR IT LI LU NL SE**

(71) Applicant: **Senju Pharmaceutical Co., Ltd.**
**5-8, Hiranomachi 2-chome, Chuo-Ku**
**Osaka-shi, Osaka 541(JP)**

(72) Inventor: **Andoh, Masako**
**Cube Mukogawa V 205, 11-24, Muko-cho**
**3-chome**
**Amagasaki-shi, Hyogo 661(JP)**
Inventor: **Ohtori, Akira**
**Cityvale New Iizuka 106, 499-1, Ohazaikisu**
**Iizuka-shi, Fukuoka 820(JP)**
Inventor: **Morita, Yasushi**
**6-10-305, Nishimidorigaoka 2-chome**
**Tayonaka-shi, Osaka 560(JP)**

(74) Representative: **von Kreisler, Alek,**
**Dipl.-Chem. et al**
**Patentanwälte Von Kreisler-Selting-Werner,**
**Deichmannhaus am Hauptbahnhof**
**W-5000 Köln 1(DE)**

(54) Microspheres for ophthalmic use.

(57) The object of the invention is to entrap a topical ophthalmic drug in a microsphere made of a high molecular compound, use it in the form of eye-drops, ophthalmic ointment, ophthalmic gel or the like, and thereby insure an effectively modulated release of the drug after topical administration.

Release of a drug is controlled by means of a microsphere made of a high molecular compound which dissolves or swells in a pH region approximating that of body fluids and containing the drug as encapsulated therein.

EP 0 490 305 A2

## BACKGROUND OF THE INVENTION

### 1. Field of the Invention

The present invention relates to a microsphere for use in, among others, pharmacotherapy in the ophthalmological field. More particularly, the invention relates to a microsphere for ophthalmic use which comprises a microsphere containing a topical ophthalmic drug and adapted to dissolve or swell in a pH region approximating the humoral pH of a recipient, which microsphere is able to keep the drug stable even when the latter is labile in aqueous environment and release the drug at a modulated rate.

The phrase "release the drug at a modulated rate" as used herein means that the microsphere inhibits release of the drug in the weakly acidic region but releases it in the physiological pH region for a desired time at a substantially zero-order rate.

### 2. Description of the Prior Art

For local administration of a drug in the ophthalmological field, several dosing schemes such as instillation of eye-drops, application of an ointment and the use of an injectable preparation are available but the instillation of a liquid preparation is the most expedient and least painful to the patient. However, for the conventional eye-drops to fully display their efficacy, they must be instilled at least twice to four times a day or, in some cases, even more frequently. Therefore, in order to increase the duration of effect of an ophthalmic drug and, hence, improve the bioavailability of the drug after instillation, studies have been undertaken on dosage forms, such as ointments, gels, inserts, etc., which would be conducive to that effect. These dosage forms may be superior to eye-drops in the duration of action but the problem of foreign body sensation in the eye, that of dislodgement from the cul-de-sac in the case of an insert, etc. remain to be resolved as yet.

Meanwhile, much research has been done into ophthalmic suspensions each comprising a drug suspended in an instillable vehicle and it is known that suspended particles are retained well in the anterior segment of the eye when the particle size is controlled at about 25 $\mu$ m [Sieg, J. W. et al., Journal of Pharmaceutical Science 69, 863-864 (1980)]. Furthermore, an ophthalmic preparation using a polylactic acid or a polycyanoalkyl acrylate as a microspherical carrier is also known (Vidmar, V. et al., Journal of Microencapsulation 2, 289-292 (1985) and Marchal-Heussler, L. et al., International Journal of Pharmaceutics 58, 115-122 (1990). However, according to these technologies, the drugs which can be formulated as ophthalmic suspensions are limited to water-insoluble drugs. Thus, if a water-soluble drug is employed, it will be quickly released from a polylactic acid or polycyanoalkyl acrylate microsphere and it is very difficult to control the release rate.

The fact that a drug entrapped in a microsphere made of a high molecular compound is generally retained stably in the microsphere even if it is an inherently unstable compound suggests that should there be available an ophthalmic microsphere made of a high molecular compound which can effectively control the release of the drug in the presence of body fluids, such as tears, it could be successfully exploited in the provision of a topical ophthalmic suspension. Furthermore, if such a microsphere made of a high molecular compound and containing a drug substance which can be administered locally to the eye be processed into an ointment or a gel, one might obtain an ophthalmic ointment or gel with a controlled kinetic profile and, hence, provide a novel ophthalmic dosage form for topical ophthalmic therapy. Therefore, development of such a microsphere made of an ophthalmologically compatible high molecular compound has been awaited in earnest by all concerned with clinical ophthalmology.

Under the circumstances, the inventors of the present invention did intensive research to develop microspheres of a high molecular compound which could effectively modulate the release of a water-soluble drug and found that when a water-soluble drug is sealed into a microsphere made of a high molecular compound which is adapted to dissolve or swell at the pH of body fluids such as tears, namely in the pH region of 4 to 8, the release of the entrapped drug could be effectively controlled and that a very beneficial ophthalmic preparation for topical application might be provided by applying the technology to pharmaceutical manufacture. The present invention has been accomplished on the basis of the above finding.

## SUMMARY OF THE INVENTION

The present invention is, therefore, directed to a microsphere containing a drug substance which can be administered locally to the eye, and made of a high molecular compound adapted to dissolve or swell in a pH region approximating the humoral pH of a recipient.

## DETAILED DESCRIPTION OF THE INVENTION

The high molecular compound which dissolves or swells in a pH region approximating the humoral pH, which is employed in the present invention, includes methyl methacrylate-butyl methacrylate-dimethylaminoethyl methacrylate copolymer, methacrylic acid-ethyl acrylate copolymer, methacrylic acid-methyl methacrylic copolymer, ethyl acrylate-methyl methacrylate-trimethylammonioethyl methacrylate chloride copolymer, methyl methacrylate-ethyl acrylate copolymer and polyvinyl acetal diethylaminoacetate, to name but a few. These compounds characteristically dissolve at pH $\leq$ 4, dissolve at pH $\geq$ 5.5, dissolve at pH $\geq$ 6, swell at pH about 4 to 8, swell at pH about 4 to 8 and dissolve at pH $\leq$ 5.8, respectively.

Since these high molecular compounds vary in the pH at which they dissolve or swell, it is possible to provide a microsphere which dissolves or swells at a desired pH by using these compounds either singly or in an appropriate combination.

As these high molecular compounds, such commercial products as Eudragit L, Eudragit S, Eudragit RS (Eudragit is a trademark and their chemical compositions will be clarified afterwards), etc. can be employed with advantage. Although these high molecular compounds dissolve or swell in a pH region approximating the humoral pH of the recipient, namely in the neighborhood of pH 4 to 8, as mentioned above and, as such, are suited for purposes of the present invention, it is advisable to employ two or more of them in combination, rather than any one of them, when dissolution or swelling in a more critically defined pH range is required.

The drug which can be administered locally to the eye, which is incorporated in the ophthalmic microsphere of the invention, may be any water-soluble drug that is topically used in the ophthalmological field and includes, among others, antiglaucoma drugs such as pilocarpine hydrochloride, timolol maleate, epinephrine, etc., anticataract drugs such as pirenoxine etc., antiinflammatory drugs such as pranoprofen, glycyrrhizinic acid, water-soluble azulene, dexamethasone sodium metasulfobenzoate etc., antiallergic drugs such as amlexanox, sodium cromoglycate, etc., antihistaminics such as chlorpheniramine maleate, diphenhydramine hydrochloride, etc., antiviral drugs such as idoxuridine etc., and antibacterial agents such as gentamicin, cefmenoxime hydrochloride, sulbenicillin sodium and so on.

The ophthalmic microsphere of the present invention can be manufactured by any of the techniques used commonly in the manufacture of microspheres. Thus, any of spray-drying, coacervation, drying-in-liquid and other methods can be utilized. For detailed discussions on these methods, reference may be had to, inter alia, Kondo, T. and Koishi, M.: Sinpan Microcapsule (1987; Sankyo Shuppan K.K.). More specifically, the O/O-, O/W- and W/O/W drying-in-liquid methods can be recommended as particularly useful procedures but any other appropriate method for manufacture of microspheres can also be employed.

The ophthalmic microsphere of the present invention which can be obtained in the above manner usually has a mean particle size of about 1 to about 70 $\mu$ m, although its particle size may vary depending on the kind of copolymer, the manufacturing method employed and so on. The particle size of the present ophthalmic microspheres may be preferably adjusted to about 10 to about 30 $\mu$ m.

In the manufacture of the microsphere of the present invention for ophthalmic use by means of the above technology, a drug that can be applied locally to the eye is incorporated in said microsphere made of a high molecular compound. The ratio of the drug to the high molecular compound is chosen with reference to the species or composition of the high molecular compound, the type of drug, the object of use and so on. Generally speaking, about 1 to 100 parts by weight, preferably about 5 to 50 parts by weight, of the high molecular compound is used to each weight part of the drug.

When the ophthalmic microsphere of the present invention is used as an ophthalmic suspension, the microsphere can be suspended in an aqueous vehicle to provide eye-drops. For use as an ophthalmic ointment, it is uniformly kneaded with an ophthalmic ointment base which is commonly employed. For use as an ophthalmic gel, the microsphere is uniformly dispersed in a gel base. In these preparations may be incorporated appropriate amounts of an excipient, suspending agent, pH adjusting agent, buffer, isotonizing agent and so on which are commonly used in the manufacture of ophthalmic drug products. In some cases, unless contrary to the object of the invention, other non-encapsulated medicinally effective ingredients may also be incorporated.

## EXAMPLES

**Example 1** [Microspheres containing pilocarpine hydrochloride]

Pilocarpine hydrochloride was mixed with the acrylic resins (trademark Eudragit) mentioned in Table 1, respectively, to give the 6 different compositions shown in Table 1 and each was dissolved in 20 ml of methanol. To 100 ml of cottonseed oil in which 2 g of soybean lecithin had been dissolved was added the above solution dropwise with constant stirring using a propeller stirrer. After completion of dropwise addition, the methanol was distilled off at about 40°C The residue was centrifuged and the sediment was washed with n-hexane to give pilocarpine hydrochloride-containing microcapsules having a mean particle size of 10 to 30 $\mu$ m. The six kinds of microspheres obtained in the above manner are designated No. 1 through No. 6.

The chemical compositions of the Eudragit (trademark) resins used in this and the following examples are as follows.

Eudragit L-100:     Methacrylic acid-methyl methacrylate copolymer (composition ratio = 1/1) (molecular weight = 135,000)

Eudragit S-100:     Methacrylic acid-methyl methacrylate copolymer (composition ratio = 1/2) (molecular weight = 135,000)

Eudragit RSPML:     Ethyl acrylate-methyl methacrylate-trimethylammonioethyl methacrylate chloride copolymer (composition ratio = 1/2/0.2) (molecular weight = 150,000)

Eudragit RSPM :     Ethyl acrylate-methyl methacrylate-trimethylammonioethyl methacrylate chloride copolymer (composition ratio = 1/2/0.1) (molecular weight = 150,000)

### Table 1

| No. | 1 | 2 | 3 |
|---|---|---|---|
| Pilocarpine hydrochloride | 0.05 g | 0.05 g | 0.05 g |
| Eudragit L-100 | 0.5 g | 0.4 g | 0.1 g |
| Eudragit S-100 | | | |
| Eudragit RSPML | | 0.1 g | 0.4 g |
| Eudragit RSPM | | | |

| No. | 4 | 5 | 6 |
|---|---|---|---|
| Pilocarpine hydrochloride | 0.05 g | 0.05 g | 0.05 g |
| Eudragit L-100 | | | |
| Eudragit S-100 | 0.5 g | 0.4 g | 0.1 g |
| Eudragit RSPML | | | |
| Eudragit RSPM | | 0.1 g | 0.4 g |

**Example 2** [Timolol maleate-containing microspheres]

In 20 ml of methanol were dissolved 0.05 g of timolol maleate and 0.5 g of Eudragit S-100 (trademark) and the solution was added dropwise to 100 ml of cottonseed oil, in which 2 g of soybean lecithin had been dissolved, under constant stirring with a propeller stirrer. After completion of dropwise addition, the methanol was distilled off at about 40°C and the residue was centrifuged. The sediment was washed with n-hexane to give timolol maleate-containing microspheres having a mean particle size of 10 to 30 $\mu$ m.

**Example 3** [Pirenoxine-containing microspheres]

4

In 20 ml of methanol was dissolved 0.5 g of Eudragit (trademark) and 0.01 g of pirenoxine was then uniformly dispersed in the solution. This suspension was added dropwise to 100 ml of cottonseed oil, in which 2 g of lecithin had been dissolved, under constant stirring with a propeller stirrer. The methanol was then distilled off at about 40°C and the residue was centrifuged. The sediment was washed with n-hexane to give pirenoxine-containing microspheres having a mean particle size of 10 to 30 $\mu$ m. The proportions of pirenoxine and Eudragit (trademark) used are shown in Table 2. The three kinds of microspheres thus obtained are designated No. 7 through No. 9.

Table 2

| No. | 7 | 8 | 9 |
|---|---|---|---|
| Pirenoxine | 0.01 g | 0.01 g | 0.01 g |
| Eudragit S-100 | 0.5 g | | |
| Eudragit L-100 | | 0.5 g | 0.15 g |
| Eudragit RSPM | | | 0.35 g |

**Example 4** [Pranoprofen-containing microspheres]

In 10 ml of chloroform was dissolved 0.5 g of Eudragit (trademark) RSPM. Then, 50 mg of pranoprophen was added and evenly dispersed by sonication. This dispersion was added dropwise to 100 ml of 1% aqueous solution of polyvinyl alcohol under constant stirring with a propeller stirrer. After completion of dropwise addition, the chloroform was distilled off at about 40 °C and the residue was washed with distilled water to give pranoprofen-containing microspheres having a mean particle size of 10 to 30 $\mu$ m.

**Example 5** [Amlexanox-containing microspheres]

In 2 ml of 0.1N-sodium hydroxide solution containing 200 mg of polyvinyl alcohol was dissolved 50 mg of amlexanox and the solution was added to 10 ml of methylene chloride in which 0.5 g of Eudragit RSPM (trademark) had been dissolved. The mixture was sonicated to provide an emulsion. This emulsion was added dropwise to 100 ml of 1% polyvinyl alcohol solution under stirring with a propeller stirrer. After completion of dropwise addition, the methylene chloride was distilled off at about 40°C and the residue was centrifuged. The sediment was washed with distilled water to give amlexanox-containing microspheres having a mean particle size of 10 to 30 $\mu$ m.

**Example 6** [A release test of pilocarpine hydrochloride-containing microspheres]

The microspheres prepared in Example 1 were immersed in 1/150 M phosphate buffer at pH 5 or 7 and shaken at 37°C to determine the time course of release of pilocarpine hydrochloride. The results of this in vitro release test are shown in Table 3.

## Table 3

| Release of pilocarpine hydrochloride (%) | | | | | |

| Time (hrs.) | No. 1 | | No. 2 | | No. 3 | |
|---|---|---|---|---|---|---|
| | pH 5 | pH 7 | pH 5 | pH 7 | pH 5 | pH 7 |
| 0.5 | 88.1 | 93.9 | 56.4 | 100 | 69.2 | 72.2 |
| 1.0 | 89.3 | 94.0 | 63.1 | | 100 | 100 |
| 2.0 | 89.8 | 100 | 72.7 | | | |
| 4.0 | 100 | | 79.5 | | | |

| Release of pilocarpine hydrochloride (%) | | | | | |

| Time (hrs.) | No. 4 | | No. 5 | | No. 6 | |
|---|---|---|---|---|---|---|
| | pH 5 | pH 7 | pH 5 | pH 7 | pH 5 | pH 7 |
| 0.5 | 7.5 | 22.3 | 6.2 | 11.1 | 13.4 | 18.0 |
| 1.0 | 9.6 | 29.6 | 7.2 | 13.3 | 15.7 | 19.7 |
| 2.0 | 10.8 | 43.9 | 7.1 | 20.9 | 19.4 | 21.6 |
| 4.0 | 12.5 | 53.2 | 8.9 | 24.5 | 22.3 | 22.0 |
| 24.0 | 22.8 | 61.1 | 15.1 | 55.3 | 37.3 | 43.6 |

**Example 7** [Time course of rabbit pupil diameter after instillation of pilocarpine hydrochloride-containing microspheres]

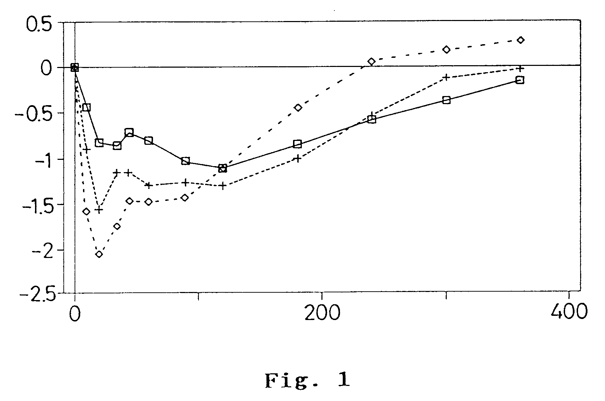

The pilocarpine hydrochloride-containing microspheres prepared in Example 1 were distilled to the rabbit eye and the time course of pupil diameter was monitored. The pilocarpine hydrochloride-containing microspheres (No. 2 and No. 4 in Example 1) were respectively suspended in phosphate buffer (pH 5) at a final concentration of 2% as pilocarpine hydrochloride. The results of this test are shown in Fig. 1. The mean residence time of activity of efficacy calculated from Fig. 1 are shown in Table 4. It is apparent that the mean residence time of activity is about 2 to 3 times as high in the microsphere groups as in the control group.

Table 4

| | Mean residence time of activity (in minutes) |
|---|---|
| Control group | 56.0 |
| Microsphere No. 2 | 123.6 |
| Microsphere No. 4 | 150.5 |

6

**Example 8** [A release test of pirenoxine-containing microspheres]

The pirenoxine-containing microspheres prepared in Example 3 were immersed in 1/150 M phosphate buffer at pH 4 or 7 and shaken at 37°C to determine the time course of release of pirenoxine. The results are shown in Table 5. At pH 7, about 30 to 77% of pirenoxine was released in 5 minutes. At pH 4, the amount of release was not more than about 30% even after 24 hours. Thus, a tendency of pH dependency was noted. The sample Nos. shown in Table 5 correspond to the sample Nos. given in Example 3.

Table 5

| Time (hrs.) | Release of pirenoxine at pH 4(%) | | |
|---|---|---|---|
| | Sample 7 | Sample 8 | Sample 9 |
| 1.0 | 17.0 | 20.6 | 9.8 |
| 2.0 | 20.0 | 21.5 | 9.8 |
| 4.0 | 22.2 | 23.2 | 9.9 |
| 24.0 | 27.6 | 29.0 | 10.9 |

**Example 9** [Stability of pirenoxine in the microsphere]

The pirenoxine-containing microspheres (No. 9) prepared in Example 3 were immersed in 1/150 M phosphate buffer at pH 4 and shaken at 40°C to estimate the stability of pirenoxine in the microspheres. The results are shown in Table 6. It is apparent that pirenoxine was still stable even after one week of continued shaking at 40°C but the pirenoxine content in the control aqueous solution had been reduced to about one-half.

Table 6

| | % Residue after 1 week at 40°C |
|---|---|
| Aqueous pirenoxine solution | 53.2% |
| Pirenoxine-containing microsphere | 99.5% |

**Example 10** [Manufacture of eye-drops-1]

Using the pirenoxine-containing microspheres (Sample No. 9) prepared in Example 3, eye-drops were manufactured according to the following formula.

| | |
|---|---|
| Pirenoxine-containing microspheres | 0.2 g |
| Boric acid | 1.8 g |
| Sodium edetate | 0.01 g |
| Polysorbate 80 | 0.05 g |
| Methyl p-hydroxybenzoate | 0.026 g |
| Propyl p-hydroxybenzoate | 0.014 g |
| Sterilized pure water | 100 ml |

The above composition was adjusted to pH 4 and 290 mOsm.

**Example 11** [Manufacture of eye-drops-2]

Using the pranoprofen-containing microspheres prepared in Example 4, eye-drops were manufactured according to the following formula.

| Pranoprofen-containing microspheres | 1.0 g |
| Sodium dihydrogen phosphate | 0.1 g |
| Sodium chloride | 0.9 g |
| Sodium edetate | 0.01 g |
| Polyvinyl alcohol | 0.1 g |
| Benzalkonium chloride | 0.005 g |
| Sterilized pure water | 100 ml |

The above composition was adjusted to pH 5 and 290 mOsm.

**Example 12** [Manufacture of an ophthalmic ointment]

To the timolol maleate-containing microspheres prepared in Example 2 were added small portions of liquid paraffin and, after kneading, white petrolatum was added and stirred well to give an ointment.

**Example 13** [Manufacture of an ophthalmic gel]

The amlexanox-containing microspheres prepared in Example 5 were added to an aqueous solution (pH 7) containing 1% of a carboxyvinyl polymer and the mixture was vigorously stirred to give a gel comprising a uniform dispersion of the microspheres.

The ophthalmic microsphere according to the present invention is very useful as a medicinal product for use in the ophthalmological field in that while release of the locally administrable ophthalmic drug contained therein is inhibited in a weakly acidic pH region, the release of the active agent is effectively controlled in a pH region approximating the physiological pH of body fluids and also in that even a drug which is unstable in aqueous environment can be retained stably in the microsphere.

**BRIEF DESCRIPTION OF THE DRAWING**

Fig. 1 shows the time course of pupil diameter of rabbits treated with the pilocarpine hydrochloride-containing microsphere of the invention as tested in Example 7, with pupil diameter (in mm) plotted on the ordinate against time (in minutes) on the abscissa.

◊ : The time course of pupil diameter in the control group

+ : The time course of pupil diameter in the group treated with Sample No. 2 prepared in Example 1.

□ : The time course of pupil diameter in the group treated with Sample No. 4 prepared in Example 1.

**Claims**

1. A pharmaceutical microsphere for ophthalmic use which comprises a microsphere containing a drug substance which can be administered locally to the eye and made of a high molecular compound adapted to dissolve or swell in a pH region approximating the humoral pH of a recipient.

2. A pharmaceutical microsphere for ophthalmic use as claimed in claim 1 wherein said high molecular compound adapted to dissolve or swell in a pH region approximating the humoral pH is one or more members selected from the group consisting of methacrylic acid-ethyl acrylate copolymer, methacrylic acid-methyl methacrylate copolymer, ethyl acrylate-methyl methacrylate-trimethylammonioethyl methacrylate chloride copolymer, methyl methacrylate-ethyl acrylate copolymer, methyl methacrylate-butyl methacrylate-dimethylaminoethyl methacrylate copolymer and polyvinyl acetal diethylaminoacetate.

3. A pharmaceutical microsphere for ophthalmic use as claimed in claim 1 or 2 wherein said drug which can be locally administered to the eye is one or more members selected from the group consisting of antiglaucoma drugs, anticataract drugs, antiinflammatory drugs, antihistaminics, antiallergic drugs, antiviral agents and antimicrobial agents.

Fig. 1